# EUROPÄISCHE PATENTSCHRIFT

(11) **EP 0 805 703 B1**
(45) Veröffentlichungstag und Bekanntmachung des Hinweises auf die Patenterteilung: **28.07.1999**
(21) Anmeldenummer: 96900273.2
(22) Anmeldetag: 10.01.1996
(51) Int. Cl.: A61M 29/02, A61M 36/12, A61N 5/10

(54) **BALLON-KATHETER ZUR VERHINDERUNG DER RE-STENOSE NACH ANGIOPLASTIE, UND VERFAHREN ZUM HERSTELLEN EINES BALLON-KATHETERS**
BALLOON CATHETER USED TO PREVENT RE-STENOSIS AFTER ANGIOPLASTY AND PROCESS FOR PRODUCING A BALLOON CATHETER
CATHETER A BALLONNET DESTINE A EVITER DES PHENOMENES DE RESTENOSE APRES ANGIOPLASTIE ET PROCEDE DE PRODUCTION D'UN CATHETER DE TYPE BALLONNET

(30) Priorität: 17.01.1995 DE 19501154
(43) Veröffentlichungstag der Anmeldung: 12.11.1997
(73) Patentinhaber: Hehrlein, Christoph, 66118 Heidelberg (DE)
(72) Erfinder: Hehrlein, Christoph, 66118 Heidelberg (DE)
(74) Vertreter: KOHLER SCHMID + PARTNER
(86) Internationale Anmeldenummer: DE9600042
(87) Internationale Veröffentlichungsnummer: WO9622121

(56) Entgegenhaltungen:
- EP-A- 0 433 011
- WO-A-93/04735
- US-A- 5 199 939
- US-A- 5 213 561

## Beschreibung

Die Erfindung betrifft einen Ballon-Katheter.

Die Angioplastie einer Gefäßenge bzw. Gefäßstenose durch Arteriosklerose mit einem Ballon-Katheter führt in 30 - 50% aller behandelten Patienten zu einer überschießenden Zellvermehrung in der Gefäßwand, die eine Re-Stenose des Gefäßes herbeiführt. Die Angioplastie löst eine überschießende Zellvermehrung in der Media der Gefäßwand sowie die Einwanderung von glatten Muskelzellen der Media in die innerste Arterienschicht aus. Dieser Prozeß führt zur sogenannten Intimahyperplasie, die das Gefäß erneut verengt.

Andere Methoden der Angioplastie mit dem Ziel, durch Laser- oder Atherektomiekatheter verschlossene Gefäße wieder zu eröffnen oder das stenotische Material zu entfernen, sind ebenfalls gebräuchlich, führen jedoch wie die Verwendung von Ballon-Kathetern zur Intimahyperplasie.

Die Verhinderung von überschießendem Zellwachstum durch radioaktive Strahlung ist durch die Behandlung von Tumoren oder die Behandlung der Intimahyperplasie hinreichend bekannt.

Eine Möglichkeit, radioaktive Strahlung zu Verhinderung der Intimahyperplasie einzusetzen, ist das vorübergehende Einbringen von radioaktiven Instrumenten oder Apparaten in die Arterie oder in einen Ballon-Katheter (Patent US 5,199,939 oder US 5,213,561). Der Nachteil dieser Methoden besteht darin, daß entweder aufgrund einer mangelnden Zentrierung eines dünnen, radioaktiven Drahtes oder Katheters die Gefäßwand nicht gleichmäßig bestrahlt wird (Patent US 5,199,939) oder kein unmittelbarer Kontakt der radioaktiven Elemente mit der Gefäßwand besteht (Patent US 5,213,561 und EP 0 633 041 A1)) und damit höhere Strahlendosen zur Verhinderung der Re-Stenose notwendig werden.

Reine Beta-Strahler wirken z.B. hauptsächlich im Nahbereich im Gewebe und die Strahlenwirkung läßt mit zunehmender Entfernung vom Ausgangsort deutlich nach. Hochenergetische Gamma-Strahlung, ausgehend z.B. von dem Radionuklid Iridium-192 (Patent US 5,213.561), penetriert zwar den Führungskatheter radioaktiver Elemente ohne größeren Dosisverlust, die Strahlungdosis wird jedoch nicht komplett in der Arterienwand aufgenommen, sondern penetriert den Körper des Patienten. Sämtliche Katheter mit radioaktiven Elementen müssen ausreichend flexibel und weich in ihrer Konsistenz beschaffen sein, um Gefäßverletzungen zu vermeiden.

Eine Möglichkeit, die Gefäßwand unmittelbar mit niedrigen Dosen zu bestrahlen, ist die Anwendung von radioaktiven Stents oder Gefäßimplantaten (Patent EP 04 33 011 A1, Patent DE 43 15 002 C1). Die Implantation von Stents kann jedoch durch das Einbringen von Fremdmaterial in die menschliche Arterie zu Blutgerinnseln und zum Gefäßverschluß führen, die in Gewebszerstörungen des zu versorgenden Organs resultieren können. Außerdem können die derzeit klinisch verwendeten Stents Fremdkörperreaktionen hervorrufen, die die Einwanderung von Entzündungszellen in die Gefäßwand nach sich zieht. Weiterhin ist zur Minimierung der Strahlenbelastung des Gesamtorganismus eine kurzfristige Strahleneinwirkung gegenüber einer langfristigen Strahlenbehandlung durch ein radioaktives Gefäßimplantat vorzuziehen.

Aus WO-A-93/04735 ist ein Katheter bekannt, der einen Ballon mit radioaktiven Elementen aufweist, die an der Oberfläche des Ballons angebracht sind. Die einzelnen radioaktiven Elemente sind streifenförmig ausgebildet und mit Abstand zueinander auf der Ballonoberfläche angebracht. Dieses Dokument offenbart darüber hinaus noch Stents, die radioaktives Material enthalten oder mit radioaktivem Material beschichtet sind. Die Stents weisen ein Gittergeflecht auf, so daß die Strahlung lokal erhöht ist.

Die Erfindung vermeidet die genannten Nachteile. Zur Erfindung gehören ein Ballon-Katheter sowie ein Verfahren zu dessen Herstellung.

Radioaktive Elemente sollten auf der Oberfläche von Ballon-Kathetern lokalisiert sein und ausreichend plastisch verformbar sein, um bei Wandunregelmäßigkeiten des Gefäßes (bei der Arteriosklerose des Menschen, in der Regel) in direktem Kontakt mit der Gefäßwand zu verbleiben. Ein wesentliches Merkmal dieser Erfindung ist, daß durch die Verformbarkeit eines Ballons aus Plastik in Verbindung mit der homogenen Verteilung der Radioaktivität auf oder an der Oberfläche die Arterienwand gleichmäßig und unmittelbar bestrahlt wird. Dies wird gewährleistet durch Radionuklide, die in der Wand des Ballons verankert sind oder festhaftend an der Ballonwand in Form eines radioaktiven Filmes angebracht sind. Die Radionuklide werden entweder direkt in die Ballonwand implantiert oder entstehen durch Beschuß eines metallischen Filmes mit geladenen Teilchen.

Ein radioaktiver Ballon-Katheter kann dazu eingesetzt werden, das verengte Gefäß gleichzeitig aufzudehnen und durch Abgabe der radioaktive Strahlung in die Arterienwand die Entwicklung einer Intimahyperplasie und damit einer erneuten Stenose zu verhindern. Die zusätzlichen Einführung eines zweiten Gerätes oder eines Gefäßimplantates zu Verhinderung der Intimahyperplasie ist dann nicht mehr notwendig. Idealerweise wird während der Aufdehnung des Ballons der Blutfluß durch den Katheter gelenkt, damit Gefäßverschlüsse bis zu 1 Stunde während der Aufdehnung des Ballons vom Patienten toleriert werden. Dies wird durch einen sogenannten Perfusions-Ballon-Katheter erreicht.

Radionuklid-Species in der Ballonwand mit vorzugsweise geringer Reichweite (z.B. Beta-Strahler) sollen verwendet werden. Radionuklide, die überwiegend Gamma-Strahlung aussenden, sind eher ungeeignet für einen radioaktiven Ballon-Katheter zur Verhinderung der Re-Stenose. Die radioaktive Strahlung wird seitlich durch den Ballon abgeben, wodurch nur die unmittelbar gedehnte Gefäßwand bestrahlt wird. Um bei homogener Aktivität der Ballonoberfläche einem Abfall der Strahlendosis an den Enden eines Ballons vorzubeugen, kann die Aktivität an den Enden lokal erhöht werden.

Weitere Merkmale und Vorteile der Erfindung ergeben sich der nachfolgenden Beschreibung von Ausführungsbeispielen der Erfindung anhand der Zeichnung, die erfindungswesentliche Einzelheiten zeigt, und aus den Ansprüchen. Es zeigen
- Fig. 1A: im Längsschnitt einen Ballon-Katheter im ungedehnten Zustand in einer Arterie,
- Fig. 1B: einen Querschnitt durch Fig. 1A,
- Fig. 2A: im Längsschnitt den Ballon-Katheter in der Arterie im gedehnten Zustand,
- Fig. 2B: einen Querschnitt durch Fig. 2A,
- Fig. 3: im Längsschnitt einen gedehnten Ballon-Katheter mit in der Ballonwand verteilten Radionukliden,
- Fig. 4: im Längsschnitt einen gedehnten Ballon-Katheter mit einem an seiner Außenwand angebrachten Polymerfilm mit einer Radionuklid-Species.

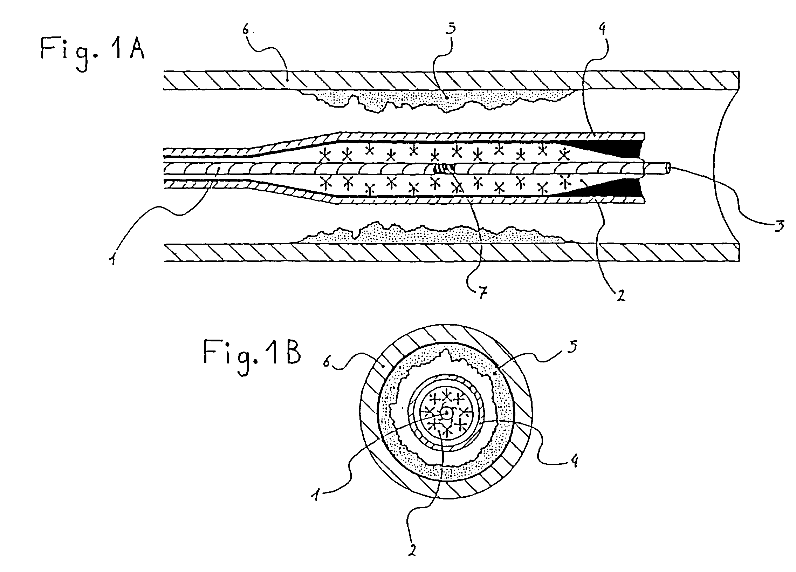

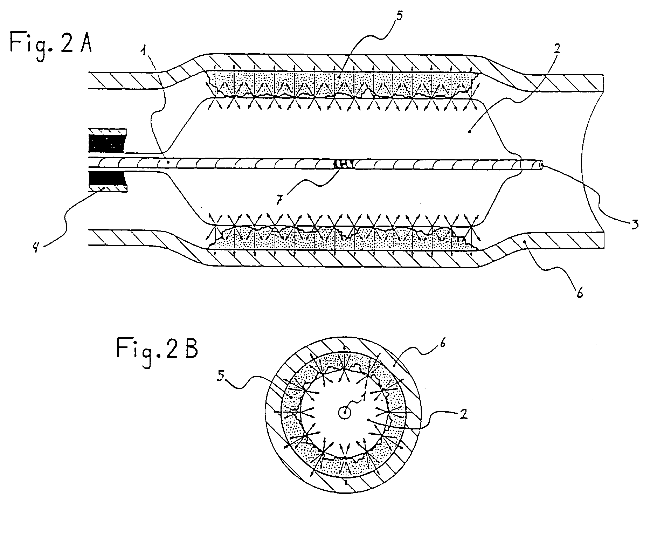

Der erfindungsgemäße Ballon-Katheter besteht aus einem Katheter 1, an dessen Spitze sich ein gefalteter, radioaktiver Ballon 2 befindet (Fig. 1A, 1B und 2A, 2B). Durch ein Lumen 3 des Katheters kann ein Führungsdraht in die Arterie geschoben werden. Um den Ballon-Katheter befindet sich eine strahlenundurchlässige, flexible Schiene 4 aus Metall, z.B. aus Blei oder einem strahlenundurchlässigen Plastik (Tefzel TM Tube). Die Dicke der Schiene 4 beträgt zwischen 0,1 und 4 mm. Diese Schiene wird am Wirkort im Bereich der Stenose 5 zurückgezogen. Sie dient dazu, die Strahlung nicht während des Einführens und des Vorschiebens des Ballon-Katheters abzugeben, sondern nur am Wirkort im Bereich der Stenose 5 in der Arterie 6. Nachdem die Strahlenschutzschiene zurückgezogen wurde, kann der Ballon im Gefäß aufgedehnt werden. Es befindet sich eine Markierung 7 am Ballon-Katheter, die die Mitte des Ballons anzeigt.

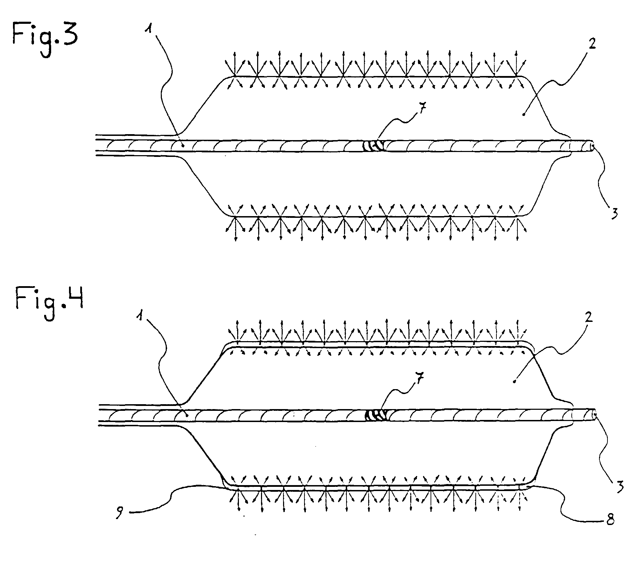

Im gedehnten Zustand (Figur 2A, 2B) liegt der Ballon an der Arterienwand an, vergrößert das Gefäßlumen und gibt gleichzeitig die Strahlung ab. Die ionisierende Strahlung wird durch die Radionuklide, die direkt in der Ballonwand verteilt sind, abgegeben (Figur 3). Alternativ dazu ist die Ballonwand mit einem Film 8 aus Metall oder Plastik beschichtet, der die Radionuklide enthält (Figur 4). Zusätzlich kann noch eine strahlendurchlässige Schutzschicht 9 vorhanden sein, die eine Kontamination des umgebenden Gewebes mit radioaktivem Material verhindert.

Die Reichweite der Strahlung im Gewebe beträgt vorzugsweise 0,05 - 10 mm. Die Radionuklide, die Alpha-, Beta-, Gamma-Strahlung oder weiche Röntgen-Strahlung abgeben, sind gleichmäßig in der Ballonwand verteilt. Die Halbwertszeit (t_{1/2}) der Radionuklid-Species oder des Radionuklidgemisches sollte zwischen 5 Stunden und 3 Jahren liegen, damit eine Lagerung des Ballon-Katheters ohne größere Aktivitätsverluste möglich ist. Als Radionuklid-Species kommen z.B. das Phosphor-32 (t_{1/2} 14,3 d) oder das Nuklid Cobalt-55 (t_{1/2} 17,8 h) in Frage, welches durch Elektroneneinfang unter Emission von weicher Röntgenstrahlung in das Nuklid Eisen-55 mit einer Halbwertszeit von 2,7 Jahren zerfällt. Diese Radionuklide wirken hauptsächlich im Nahbereich in menschlichem Gewebe, d.h. jenseits von einer Entfernung von 10 mm ist die Strahlendosis sehr gering.

Die Aktivierung von Plastikmaterialien eines Ballon-Katheters kann durch mehrere Maßnahmen erfolgen.

Die Herstellung eines radioaktiven Ballons kann dadurch erfolgen, daß mindestens eine Radionuklid-Species dem Plastik des Ballons, z.B. Polyäthylen oder Latex, vor der Herstellung des Katheters zugemischt werden (Figur 3). Die Radionuklid-Species sind Bestandteile der Plastikwand. Der radioaktive Ballon wird dann nach herkömmlicher Verfahrenstechnik am Katheter befestigt. Der Ballon kann komprimiert und expandiert werden ohne daß Radioaktivität verloren geht.

Alternativ können käuflich erwerbliche Ballon-Katheter in einer Beschleunigeranlage aktiviert werden. Es kommen alle kommerziell erwerblichen Ballon-Katheter in Frage, die für eine Angioplastie zugelassen sind. Dabei wird die Wand des Ballons im expandierten Zustand mit mindestens einem Radionuklid beschossen. Eine Ionenquelle liefert die erforderliche Radionuklid-Species. Die Radionuklid-Species wird in die Beschleunigeranlage eingeschleust und wirkt als lokalisierter Ionenstrahl auf das Plastik ein. Die Matrix des Plastiks nimmt die Radionuklid-Species auf. Eine gleichmäßige Aktivierung der Ballonoberfläche wird durch Drehungen des Ballons erreicht. Um z.B. den reinen Beta-Strahler Phosphor-32 selektiv in die Ballonwand zu implantieren, wird er vorher in einer Beschleunigeranlage unter Verwendung eine Massenseparators von Phosphor-31 getrennt. Es können sämtliche kommerziell erwerbliche Ballon-Katheter aktiviert werden, auch sogenannte Perfusionskatheter mit mehreren Löchern oder Lumina vor und hinter dem Ballon, um den Blutfluß während der Aufdehnung des Ballons zu gewährleisten.

Alternativ kann ein Polymer-Film mit mindestens einer Radionuklid-Species 8 festhaftend und nicht löslich an der Außenwand eines Ballon-Katheters angebracht werden oder eine zweite, dünnere Plastikschicht 9 wird über dem radioaktiven Polymer-Film befestigt. Diese Plastikschicht ist strahlendurchlässig und verhindert die Auflösung des Filmes durch den Kontakt mit Blut oder die Ablösung des Filmes bei der Aufdehnung des Ballons (Figur 4).

Nachfolgend werden beispielhafte erfindungsgemäße Verfahren zur Herstellung eines radioaktiven Ballon-Katheters beschrieben.

### Beispiel 1

Die Reichweite der Strahlung im Gefäß ausgehend von einem radioaktiven Ballon-Katheter sollte vorzugsweise 0,05 - 10 mm betragen. Radionuklide, die Beta-Strahlung oder weiche Röntgen-Strahlung abgeben, wirken im Nahbereich in der Arterie und sind daher am besten geeignet als Bestandteile der Ballonwand oder einer Ballon-Beschichtung. Die Aktivierung von käuflich erwerbbaren Ballon-Kathetern erfolgt durch eine direkte Implantation von Radionukliden in die Ballonwand. Der Ballon wird dabei im gedehnten Zustand in einer Beschleunigeranlage mit Radionukliden beschossen. Eine gleichmäßige Aktivierung der Ballonoberfläche wird durch Drehungen des Ballons erreicht. Das Radionuklid wird im Plastikmaterial der Ballonwand aufgenommen. Um z.B. den reinen Beta-Strahler Phosphor-32 selektiv in die Ballonwand zu implantieren, wird er vorher in einer Beschleunigeranlage unter Verwendung eine Massenseparators von Phosphor-31 getrennt.

### Beispiel 2

Eine metallische Schicht, vorzugsweise aus Aluminium, Silber oder Edelstahl wird auf den gedehnten Ballon eines käuflich erwerbbaren Ballon-Katheters aufgedampft. Die Schicht sollte zwischen 2 nm und 5 µm dick sein. Anschließend erfolgt zur besseren Verankerung der metallischen Schicht mit dem Plastikmaterial ein Beschuß mit Argon-Ionen. Desweiteren werden nun entweder eine ausgewählte Radionuklid-Species direkt in diese metallische Schicht implantiert oder die metallische Schicht wird mit geladenen Teilchen, z.B. Protonen beschossen. Der Teilchenbeschuß führt zu einer Konversion von Metall-Ionen in Radionuklide, die in der Metallschicht haften.

## Patentansprüche

1. Ballon-Katheter (1), der mindestens eine mit der Ballonwand verbundene Radionuklid-Species aufweist, um Gefäßverengungen bzw. Stenosen zu beseitigen und die Re-Stenose in Arterien (6), Venen oder in Gefäßimplantaten zu verhindern oder das Wachstum von Tumoren zu vermindern, dadurch gekennzeichnet, daß die mindestens eine Radionuklid-Species in der Weise dem Plastik der Ballonwand zugemischt ist oder direkt in die Ballonwand implantiert ist oder als festhaftender Film (8) an der Ballonwand angebracht ist, daß die Ballonoberfläche eine homogene Aktivität aufweist.

2. Ballon-Katheter nach Anspruch 1, dadurch gekennzeichnet, daß die Aktivität der Radionuklid-Species an den Enden des Ballons (2) lokal erhöht ist.

3. Ballon-Katheter nach Anspruch 1 oder 2, dadurch gekennzeichnet, daß die Radionuklid-Species Alpha-, Beta-, Gamma-Strahlung oder weiche Röntgen-Strahlung emittiert und die Halbwertszeit der Strahlung mindestens 5 Stunden und längstens 3 Jahre beträgt.

4. Ballon-Katheter nach einem der Anpsrüche 1 bis 3, dadurch gekennzeichnet, daß die Radionuklid-Species eine überwiegende Nahwirkung von 0,05-10 mm vorzugsweise in menschlichem Gewebe hat.

5. Ballon-Katheter nach einem der Ansprüche 1 bis 4, dadurch gekennzeichnet, daß die Aktivität der Radionuklid-Species zwischen 0,001 mCi und 10 000 mCi pro cm Länge des Ballons (2), vorzugsweise zwischen 0,01 mCi und 10 000 mCi pro cm Länge des Ballons (2) beträgt.

6. Ballon-Katheter nach einem der Ansprüche 1 bis 5, dadurch gekennzeichnet, daß zusätzlich eine Schiene (4) aus Metall, z.B. Blei, oder strahlenundurchlässigem Plastik oder anderem strahlenundurchlässigen Material zur Strahlenprotektion den Ballon (2) im zusammengefalteten Zustand umgibt und vor der Ballon-Aufdehnung zurückgezogen werden kann.

7. Ballon-Katheter nach einem der Ansprüche 1 bis 6, dadurch gekennzeichnet, daß auf die Ballonwand ein metallischer Film (8), z.B. aus Aluminium oder Silber, aufgebracht oder in der Ballonwand verankert ist, der mindestens eine Radionuklid-Species aufweist.

8. Ballon-Katheter nach einem der Ansprüche 1 bis 6, dadurch gekennzeichnet, daß der Ballon (2) mit einem festhaftenden, nichtlöslichen Polymer-Film beschichtet ist, der mindestens eine Radionuklid-Species enthält, oder daß ein radioaktiver Film mit einer zweiten, nicht-löslichen, dünnen Schutzschicht (9) aus Plastik oder Metall bedeckt ist, die strahlendurchlässig ist.

9. Ballon-Katheter nach einem der Ansprüche 1 bis 8, dadurch gekennzeichnet, daß er mehrere Lumina oder Löcher vor und hinter dem Ballon besitzt, die den Blutfluß im Gefäß während der Aufdehnung des Ballons (2) aufrechterhalten.

10. Ballon-Katheter nach einem der Ansprüche 1 bis 9, dadurch gekennzeichnet, daß das Plastik des Ballons (2), in das mindestens eine Radionuklid-Species eingebaut wird, Polyäthylen oder Latex ist.

11. Ballon-Katheter nach einem der Ansprüche 1 bis 10, dadurch gekennzeichnet, daß mindestens eine der verwendeten Radionuklid-Species ein Beta-Strahler wie das Phosphor-32 ist.

12. Verfahren zum Herstellen eines Ballon-Katheters (1) nach einem der vorhergenden Ansprüche, der mindestens eine mit der Ballonwand verbundene Radionuklid-Species aufweist, um Gefäßverengungen bzw. Stenosen zu beseitigen und die Re-Stenose in Arterien (6), Venen oder in Gefäßimplantaten zu verhindern oder das Wachstum von Tumoren zu vermindern, wobei die mindestens eine Radionuklid-Species in der Weise dem Plastik der Ballonwand zugemischt wird oder direkt in die Ballonwand implantiert wird oder als festhaftender Film (8) an der Ballonwand angebracht wird, daß die Ballonoberfläche eine homogene Aktivtät aufweist.

13. Verfahren nach Anspruch 12, dadurch gekennzeichnet, daß auf die Ballonwand ein metallischer Film, z.B. aus Aluminium oder Silber aufgebracht oder in der Ballonwand verankert wird, der mit mindestens einer Radionuklid-Species versehen wird.

14. Verfahren nach Anspruch 12 oder 13, dadurch gekennzeichnet, daß ein Angioplastie-Katheter in einer Beschleunigeranlage unter Verwendung einer Ionenquelle radioaktiv verändert wird.

15. Verfahren nach Anspruch 12 oder 13, dadurch gekennzeichnet, daß ein Ballonkatheter (1) mit mehreren Lumina oder Löchern vor und hinter dem Ballon (2), die den Blutfluß im Gefäß während der Aufdehnung des Ballons (2) aufrechterhalten, sogenannter Perfusions-Ballon-Katheter, radioaktiv verändert wird.

## Claims

1. Balloon catheter (1) having at least one radioactive nuclear species connected to the wall of the balloon to eliminate narrowing of blood vessels or stenoses and to prevent restenosis in arteries (6), veins or in vessel implants or to reduce the growth of tumors,
characterized in that the at least one radioactive nuclear species is mixed into the plastic of the wall of the balloon or is directly implanted into the wall of the balloon or is introduced as a firmly bonding film (8) to the wall of the balloon in such a fashion that the surface of the balloon has a homogeneous activity.

2. Balloon catheter according to claim 1, characterized in that the activity of the radioactive nuclear species is locally increased at the ends of the balloon (2).

3. Balloon catheter according to claim 1 or 2, characterized in that the radioactive nuclear species emits alpha, beta, gamma radiation or soft X-ray radiation with a radiation half life of at least 5 hours and at most 3 years.

4. Balloon catheter according to any one of claims 1 to 3, characterized in that the radioactive nuclear species has a substantially short range effect of 0.05 - 10 mm, preferentially in human tissue.

5. Balloon catheter according to any one of the claims 1 through 4, characterized in that the activity of the radioactive nuclear species assumes values between 0.001 mCi and 10000 mCi per cm length of the balloon (2), preferentially between 0.01 mCi and 10000 mCi per cm of length of the balloon (2).

6. Balloon catheter according to any one of the claims 1 through 5, characterized in that an additional sheath (4), made from metal e.g. lead or a radiation impermeable plastic or another radiation impermeable material, surrounds the balloon in its folded together state for radiative protection and can be pulled back prior to expansion of the balloon.

7. Balloon catheter according to any one of the claims 1 to 6, characterized in that a metallic film (8) having the at least one radioactive nuclear species, e.g. made from aluminum or silver, is introduced onto the wall of the balloon or is anchored in the wall of the balloon.

8. Balloon catheter according to any one of the claims 1 to 6, characterized in that the balloon (2) is coated with a firmly bonding non-soluble polymer film containing the at least one radioactive nuclear species or a radioactive film is covered with a second non-soluble thin protective layer (9) made from plastic or metal which is radiation permeable.

9. Balloon catheter according to any one of the claims 1 to 8, characterized in that it has a plurality of lumina or holes upstream and downstream of the balloon to maintain blood-flow in the vessel when the balloon (2) is expanded.

10. Balloon catheter according to any one of the claims 1 to 9, characterized in that the plastic of the balloon (2) in which the at least one radioactive nuclear species is incorporated, is polyethylene or latex.

11. Balloon catheter according to any one of the claims 1 to 10, characterized in that at least one of the radioactive nuclear species is a beta-radiator such as phosphorous-32.

12. Method for the production of a balloon catheter (1) according to any one of the preceding claims having at least one radioactive nuclear species connected to the wall of the balloon for removing narrowings in blood vessels or stenoses and to prevent restenosis in arteries (6) , veins or in vessel implants or to reduce the growth of tumors, wherein the at least one radioactive nuclear species is mixed into the plastic of the wall of the balloon or directly implanted into the balloon wall or introduced onto the balloon wall in the form of a firmly bonding film (8) in such a fashion that the surface of the balloon has a homogeneous activity.

13. Method according to claim 12, characterized in that a metallic film, e.g. made from aluminum or silver and having at least one radioactie nuclear species is introduced onto the wall of the balloon or is anchored in the wall of the balloon.

14. Method according to claim 12 or 13, characterized in that an angioplasty catheter is radioactively transformed in an accelerator installation using an ion source.

15. Method according to claim 12 or 13, characterized in that a balloon catheter (1) having a plurality of lumina or holes upstream and downstream of the balloon (2) which maintain blood-flow in the vessel when the balloon (2) is expanded, a so-called perfusion balloon catheter, is radioactively changed.

## Revendications

1. Cathéter à ballonnet (1) qui comporte au moins une espèce de radionuclétide liée à la paroi du ballonnet pour supprimer des rétrécissements vasculaires ou des sténoses et pour éviter la resténose dans des artères (6), des veines ou dans des implants vasculaires, ou pour diminuer la croissance de tumeurs, caractérisé en ce que la ou les espèces de radionucléides sont mélangées à la matière plastique de la paroi du ballonnet ou sont implantées directement dans la paroi du ballonnet ou sont appliquées sur la paroi du ballonnet sous forme de film (8) adhérant solidement, de telle manière que la surface du ballonnet présente une activité homogène.

2. Cathéter à ballonnet selon la revendication 1, caractérisé en ce que l'activité de l'espèce de radionucléide est augmentée localement aux extrémités du ballonnet (2).

3. Cathéter à ballonnet selon la revendication 1 ou 2, caractérisé en ce que l'espèce de radionucléide émet un rayonnement α, β ou γ ou un rayonnement X mou et la période du rayonnement est d'au moins 5 h et d'au plus 3 ans.

4. Cathéter à ballonnet selon l'une des revendications 1 à 3, caractérisé en ce que l'espèce de radionucléide a un effet principalement de courte portée de 0,05 à 10 mm, de préférence dans le tissu humain.

5. Cathéter à ballonnet selon l'une des revendications 1 à 4, caractérisé en ce que l'activité de l'espèce de radionucléide est de 0,001 mCi à 10 000 mCi par cm de longueur du ballonnet (2), de préférence de 0,01 mCi à 10 000 mCi par cm de longueur du ballonnet (2).

6. Cathéter à ballonnet selon l'une des revendications 1 à 5, caractérisé en ce que, en outre, une gouttière (4) en métal, par exemple en plomb, ou en matière plastique imperméable aux rayonnements ou en un autre matériau imperméable aux rayonnements entoure le ballonnet (2) à l'état replié pour la protection contre les rayonnements et peut être retirée avant la dilatation du ballonnet.

7. Cathéter à ballonnet selon l'une des revendications 1 à 6, caractérisé en ce qu'un film métallique (8), par exemple en aluminium ou en argent, qui comporte au moins une espèce de radionucléide, est appliqué sur la paroi du ballonnet ou est ancré dans la paroi du ballonnet.

8. Cathéter à ballonnet selon l'une des revendications 1 à 6, caractérisé en ce que le ballonnet (2) est recouvert d'un film de polymère non soluble adhérant solidement, qui contient au moins une espèce de radionucléide, ou en ce qu'un film radioactif est recouvert d'une deuxième couche protectrice (9) mince non soluble en matière plastique ou en métal qui est perméable aux rayonnements.

9. Cathéter à ballonnet selon l'une des revendications 1 à 8, caractérisé en ce qu'il possède devant et derrière le ballonnet plusieurs lumières ou trous qui maintiennent la circulation du sang dans le vaisseau pendant la dilatation du ballonnet (2).

10. Cathéter à ballonnet selon l'une des revendications 1 à 9, caractérisé en ce que la matière plastique du ballonnet (2) dans laquelle est incorporée au moins une espèce de radionucléide est du polyéthylène ou un latex.

11. Cathéter à ballonnet selon l'une des revendications 1 à 10, caractérisé en ce qu'au moins l'une des espèces de radionucléides utilisées est un émetteur β comme le phosphore 32.

12. Procédé de production d'un cathéter à ballonnet (1) selon l'une des revendications précédentes, qui comporte au moins une espèce de radionucléide liée à la paroi du ballonnet, pour supprimer des rétrécissement vasculaires ou des sténoses et pour éviter la resténose dans des artères (6), des veines ou dans des implants vasculaires ou pour diminuer la croissance de tumeurs, où la ou les espèces de radionucléides sont mélangées à la matière plastique de la paroi du ballonnet ou sont implantés directement dans la paroi du ballonnet ou sont appliquées sur la paroi du ballonnet sous forme de film (8) adhérant solidement, de telle manière que la surface du ballonnet présente une activité homogène.

13. Procédé selon la revendication 12, caractérisé en ce qu'un film métallique, par exemple en aluminium ou en argent, qui est muni d'au moins une espèce de radionucléide, est appliqué sur la paroi du ballonnet ou est ancré dans la paroi du ballonnet.

14. Procédé selon la revendication 12 ou 13, caractérisé en ce qu'un cathéter pour angioplastie est modifié radioactivement dans une installation de type accélérateur au moyen d'une source d'ions.

15. Procédé selon la revendication 12 ou 13, caractérisé en ce qu'un cathéter à ballonnet (1) comportant devant et derrière le ballonnet (2) plusieurs lumières ou trous qui maintiennent la circulation du sang dans le vaisseau pendant la dilatation du ballonnet (2), appelé cathéter à ballonnet pour perfusion, est modifié radioactivement.
